# EUROPEAN PATENT SPECIFICATION

(11) **EP 4 132 602 B1**
(45) Date of publication and mention of the grant of the patent: **07.10.2026**
(21) Application number: 21719975.1
(22) Date of filing: 02.04.2021
(51) Int. Cl.: A61L 27/36, A61L 27/52

(54) **HYDROGEL FROM BOVINE PERICARDIUM FOR MEDICAL USE**
HYDROGEL AUS RINDERPERICARDIUM ZUR MEDIZINISCHEN VERWENDUNG
HYDROGEL PROVENANT DE PÉRICARDE BOVIN POUR UN USAGE MÉDICAL

(30) Priority: 09.04.2020 IT 202000007567
(43) Date of publication of application: 15.02.2023
(73) Proprietor: Tissuegraft S.r.l., 28100 Novara (NO) (IT); Passalacqua Escavazioni S.r.l., 14034 Castello di Annone (AT) (IT)
(72) Inventor: BOCCAFOSCHI, Francesca, Novara (NO) (IT); CALVO CATOIRA, Marta, Novara (NO) (IT); FUSARO, Luca, Novara (NO) (IT)
(74) Representative: Fiammenghi, Eva
(86) International application number: PCT/IB2021/052779
(87) International publication number: WO 2021/205309

(56) References cited:
- US-A1- 2005 124 560
- CLAUDIO-RIZO JESÚS A ET AL: "Influence of residual composition on the structure and properties of extracellular matrix derived hydrogels", MATERIALS SCIENCE AND ENGINEERING C, ELSEVIER SCIENCE S.A, CH, vol. 79, 18 May 2017 (2017-05-18), pages 793 - 801, XP085073347, ISSN: 0928-4931, DOI: 10.1016/J.MSEC.2017.05.118
- W. A. NAIMARK ET AL: "HMDC crosslinking of bovine pericardial tissue: a potential role of the solvent environment in the design of bioprosthetic materials", JOURNAL OF MATERIALS SCIENCE: MATERIALS IN MEDICINE, vol. 6, no. 4, 1 April 1995 (1995-04-01), United States, pages 235 - 241, XP055755532, ISSN: 0957-4530, DOI: 10.1007/BF00146862
- MICHAEL MEYER: "Processing of collagen based biomaterials and the resulting materials properties", BIOMEDICAL ENGINEERING ONLINE, vol. 18, no. 1, 18 March 2019 (2019-03-18), XP055609740, DOI: 10.1186/s12938-019-0647-0

## Description

### Field of the art

The present invention refers to the medical field. More in detail the present invention regards a type of hydrogel based on collagen from decellularized bovine pericardium for use as medication and especially for use as filler in treating osteoarthritis and is defined by the appended claims.

### Prior art

Hydrogels are materials formed by a three-dimensional mesh derived from natural or synthetic polymers and they were the first biomaterials developed for human use¹. Their particular nature is the capacity to absorb and preserve great quantities of water. The hydrogels are viscoelastic polymer structures with physical structures similar to those of the physiological tissues, the network that forms them is given by crosslinked polymers with covalent and noncovalent bonds. The hydrogels can be classified as natural or synthetic hydrogels, depending on the materials used for synthesizing them, or as chemical and physical hydrogels, based on the methods used for synthesizing them. The hydrogels have many characteristics that render them highly used in research, and among these interesting qualities we find the wide versatility due to the fact that their physical and chemical properties can be controlled during synthesis in order to create the hydrogel suitable for the final application. Their singular capacity to form three-dimensional meshes confers thereto the possibility of diffusing molecules and cells and, not least, their characteristics similar to those of physiological tissues render them very interesting in biomedical field². Among these characteristics, there are: biocompatibility, biodegradability, low cytotoxicity and the possibility to create injectable hydrogels.

The hydrogels have been widely studied for their biomedical applications, which include: administration of drugs (drug delivery), three-dimensional cell cultures, tissue substitutions, tissue regeneration, contact lenses and uses in sanitary products. In particular, hydrogels are already used in medication of wounds as debriding agents or in combination with other materials for producing composites more suitable to the wound type, and their highly porous structure has made them interesting for drug release, so that they are already used for topical administration or for iontophoretic administration³. Finally, they are used in the field of tissue engineering for the creation of tissue scaffolds and culture systems for the cells⁴.

The hydrogels of natural origin finely mimic the extracellular matrix, since both are three-dimension polymer networks with viscoelastic characteristics which can accommodate and support the cells. The extracellular matrix is the tissue component assigned to support the cells, it is mainly composed of collagen, and other components are elastin, fibrin, fibronectin, laminin, hyaluronic acid, proteoglycans and glycoproteins.

Among the materials used for producing natural hydrogels we have:
- those proteic: collagen, elastin, gelatin, fibrin and fibroin.
- those polysaccharide: alginic acid, hyaluronic acid and chitosan.

These materials confer to the hydrogels of natural origin advantages such as a good biocompatibility, bioactivity⁵ and a low cytotoxicity. Nevertheless, the natural hydrogels have several limitations, such as the weak mechanical properties, the unpredictability of the results due to the high variability among the batches during production⁶ which causes difficulties during their characterization, and finally, as explained above, the quick degradability.

Several of these biomaterials are derived from decellularized animal tissues; the materials mainly derive from secondary products of the food and farming industry.

The decellularization can be carried out on any tissue with chemical, physical or enzymatic methods. The best method for exploiting the decellularized tissues is that of solubilizing them for the preparation of the hydrogel, and this occurs with two main processes: the tissue is first solubilized into monomers, then a neutralization occurs, dependent on the temperature or on the pH, which stimulates the formation of new intramolecular bonds between the monomers. The main method used is that of solubilization with pepsin. During these two processes, several parameters can be modified, such as the solubilization time, so as to obtain a defined concentration of matrix, and this confers different rheological properties in the obtained hydrogels ⁷.

The objective of the decellularization of the tissues or of the organs is that of imitating the native tissues and providing an environment that is suitably recognized by the cells, and this is obtained by removing the cells but preserving the micro- and macro-architecture of the ECM and the multiproteic niche suitable for maintaining the functional vitality of the cells.

In order to obtain an optimal decellularized matrix, therefore, many factors must be considered, including the origin of the tissue, the preparation procedures and the effectiveness of the decellularization. In addition, other factors like the use of chemical crosslinking agents or the final sterilization must be under control, since they could affect the biological properties of the matrix. Specific characteristics such as the cellular density, the density of the matrix, the geometry, the thickness of the tissue and the shape must be considered in order to process it with the optimal decellularization method. In order to preserve the microstructure/nanostructure of the matrix (if requested), the composition and the biological properties, the decellularization method must be rationally selected and it must be scientifically justified for each type of matrix or organ. The critical point is not only that of using the optimal exposure of the physical forces, but also the selection of non-physiological chemical and biological agents such as detergents and enzymes⁸.

In order to summarize the main aspects of the production of a hydrogel, table 1 quantifies the ease level in terms of obtaining the material from its original source, its biocompatibility and its transformation into a hydrogel.

### Table 1

Comparison between type of material of natural origin, biocompatibility and difficulty of processing of different biomaterials. ⁹

| **Material** | **Origin** | **Biocompatibility** | **Processability** |
|---|---|---|---|
| Collagen | + | +++ | ++ |
| Elastin | + | +++ | + |
| Fibrin | +++ | ++ | + |
| Gelatin | ++ | +++ | ++ |
| Silk fibroin | + | ++ | + |
| Glycosaminoglycans | ++ | +++ | + |
| Alginate | ++ | +++ | + |
| Chitosan | ++ | + | + |
| Decellularized tissues | +++ | +++ | + |

| | | | |
|---|---|---|---|
| The score ranges from + (low) to +++ (high with reference to the simplicity of use) | | | |

Before entering into the details of the following description of the invention, one of the problems for which it is aimed is summarized in its main aspects hereinbelow. Specifically, the product according to the present invention first of all aims to be adapted as an optimal solution via intra-articular infiltration. On such matter, it is of interest to report that osteoarthritis (OA) is the most common skeletal muscular disturbance that hits both small and large articulations such as the hand, hip and the knee, which are the most affected areas. Among all the affected articulations, the OA of the knee has a prevalence of about 10% in men and 13% in women of age older than 60 ¹⁰. The OA knee has high economic and social costs and it can have a devastating impact on the quality of life of patients ¹¹. The articulations hit by osteoarthritis have a complex array of structural, tissue, cellular and biochemical changes. The inflammation mediators are over-expressed, such as IL-1α, IL-1β, TNF-β and IL-6, and in turn these activate enzymes which lead to the progressive degradation of the extracellular matrix (ECM), including collagen¹². Recently, it was demonstrated that the level of severity of the osteoarthrosis of the knee is correlated with the imbalance of the macrophages aM1 / M2 which was shown to be considerably higher in the OA of the knee than in the healthy control¹³.

In addition, the inhibitory effect of the macrophage M1 in the OA was demonstrated, on the chondrogenic differentiation of the MSC¹⁴. The biomaterials used up to present can induce a local inflammatory response¹⁵ but it is clear that the modulation of the inflammatory environment in an OA articulation, inducing the macrophage phenotype M2, is vital for maintaining and for obtaining an effective repair.

The hydrogels provides a regenerative medicine platform that is interesting for its capacity to create an environment that supports both transplanted cells and endogenous cells¹⁶. The exogenous administration of collagen can be useful for compensating for the inflammatory imbalance towards M1 which characterizes the OA. In addition, the collagen hydrogel is particularly interesting due to its similarity with the extracellular matrix (ECM).

CLAUDIO-RIZO JESÙS A ET AL: "Influence of residual composition on the structure and properties of extracellular matrix derived hydrogels", MATERIALS SCIENCE AND ENGINEERING C, vol. 79, 18 May 2017, p 793-801, discloses a process for preparing a hydrogel useful in tissue engineering and regenerative medicine comprising a) providing a decellularized and lyophilized bovine pericardium; b) hydrolysing the tissue with a 0.01 M HCl solution of pepsin and c) treating the material with water. The hydrogel produced also comprises laminin and fibronectin but the presence of elastin is not disclosed.

W. A. Naimark ET AL: "HMDC crosslinking of bovine pericardial tissue: a potential role of the solvent environment in the design of bioprosthetic materials", Journal of Materials Science: Materials in Medicine, vol. 6, no. 4, 1 April 1995, p 235-241, discloses the crosslinking of bovine pericardial tissue; it is not disclosed that the tissue is decellularized, lyophilized, grinded and enzymatically digested before the crosslinking.

Michael Meyer: "Processing of collagen based biomaterials and the resulting materials properties", BioMedical Engineering OnLine, vol. 18, no. 1, 18 March 2019, discloses the importance of the presence of elastin to retain biological features that are essential in tissue regeneration and highlights the possibility that elastin could be retained after decellularization treatments.

Therefore, the object of the present industrial invention patent application is a particular type of hydrogel which, given its specific composition, is particularly and effectively adapted for the applications thereof, especially for use in treating osteoarthritis.

### Description of the invention

The present description refers to a particular product represented by a hydrogel obtained following the decellularization of the bovine pericardium. The present description also refers to the particular process for obtaining said product.

More in detail according to the present invention, the synthesis of the hydrogel based on collagen is performed starting from the decellularized and lyophilized pericardium as raw material. After a suitable decrease of the dimensions of the material, this is enzymatically digested with pepsin in acidic solution in order to obtain a pregel.

Still more in detail, the product according to the present invention is a hydrogel based on collagen from bovine pericardium in a concentration comprised between 0.2% and 0.8% by weight.

Said product has a complex matrix in which various proteins of the extracellular matrix are preserved. The most abundant is certainly collagen (about 95%) in its various forms.

Also present, even if in smaller percentages (1-3%), is elastin, it too present in various isoforms. Other proteins of the extracellular matrix are present even if in smaller and slightly variable percentages depending on the production batches. The biological effect is nevertheless maintained, conferring to the matrices an exceptional biocompatibility and unique properties relative to the maintenance of the monocyte vitality and, still more important for the purpose of use in the context of tissue regeneration, the intrinsic ability of polarizing the macrophages massively into M2, i.e. into the phenotype which accompanies the regeneration of the tissues, turning off the inflammatory response (due instead to the macrophages polarized into M1).

It is possible to enrich the hydrogel with crosslinking agents such as: hexamethylene diisocyanate (HMDI) at concentrations variable between 0.01 and 0.1% by weight with respect to the solid content. The addition of the crosslinking agent stabilizes the gelatinous matrix and prolongs the dissolution times thereof in the fluids. Based on the use needs, it is also possible to enrich the matrix with bioactive molecules such as antibiotics, growth factors, etc. The hydrogel from decellularized bovine pericardium according to the present invention is adapted to be used as:
- hydrogel injectable via intra-articular infiltrations (as presently hyaluronic acid is used, regarding which however the same intrinsic regenerative properties are not recognized)
   - i.e. without adding anything to induce the polarization, while for the hydrogel according to the present invention this occurs spontaneously, as described below, in terms of polarization of the monocytes/macrophages and the maintenance of the vitality thereof for prolonged time periods;
- filler for treating skin ulcers, also as matrix useful for neoangiogenesis (e.g. important in diabetic foot);
- 3D matrix which allows extending microphage survival by polarizing them into M2 (delivery system to be used for example in combination with the filters for isolating the monocytes from peripheral blood);
- for use in cosmetic field;
- for use in esthetic and maxillofacial surgery.

Possible disadvantages in the production of the hydrogels consist of the imperfect superimposability of the batches in terms of percentages of the different contained proteins. Nevertheless, in the course of the experiments and studies pertaining to the present invention, it was verified that the variability does not significantly interfere with the biological activity, which is preserved, maintaining high capacities of tissue regeneration, also in terms of effectiveness of polarization of the macrophage fraction.

Hereinbelow, a hydrogel will be described that is based on collagen from bovine pericardium, obtainable with a process which provides that:
- said hydrogel is produced starting from decellularized and lyophilized pericardium;
- it is subsequently finely divided so as to reduce the dimensions thereof;
- it is enzymatically digested with pepsin in acidic solution up to obtaining a pregel;
- said pregel is treated with water for obtaining the hydrogel;
- the hydrogel thus obtained is enriched with a crosslinking agent at a concentration comprised between 0.01% and 0.1% by weight with respect to the content of the solid, and in which said crosslinking agent is, by way of a non-limiting example, hexamethylene diisocyanate.

### Description of the figures

The present description will be described in detail hereinbelow with reference to the enclosed figures which indicate interesting results that confirm the properties of the hydrogels according to the present invention in sustaining and optimizing the regeneration of the tissues. In detail:

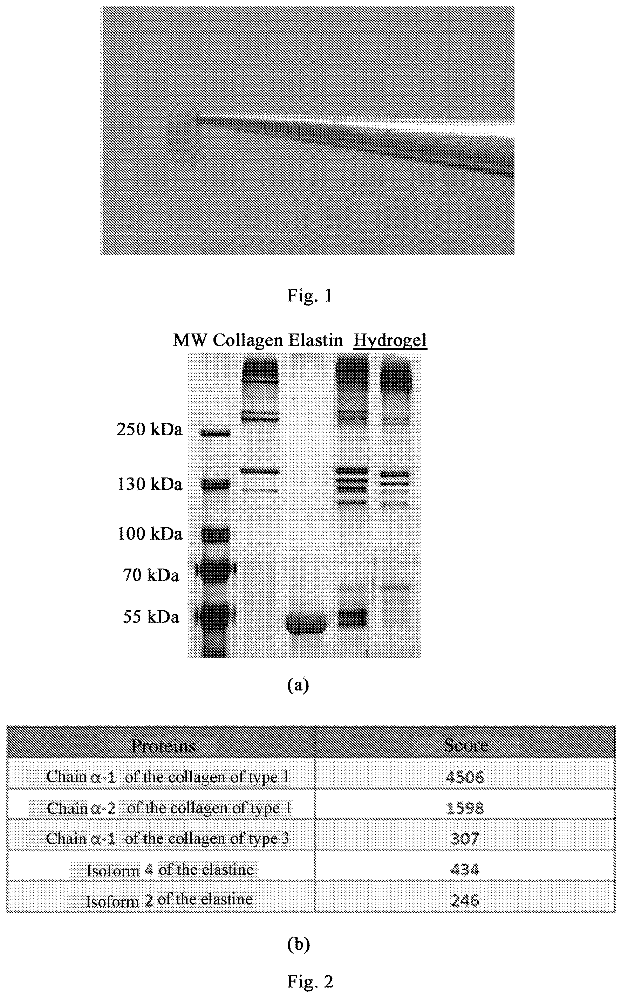

FIGURE 1 shows the macroscopic image of the hydrogel from decellularized bovine pericardium according to the present invention.
FIGURE 2 shows the data relative to the protein content compared with the collagen and elastin standards (fig. 2(a)), and proteomic analysis of the collagen and elastin isoforms present in a sample of hydrogel (fig.2(b)).

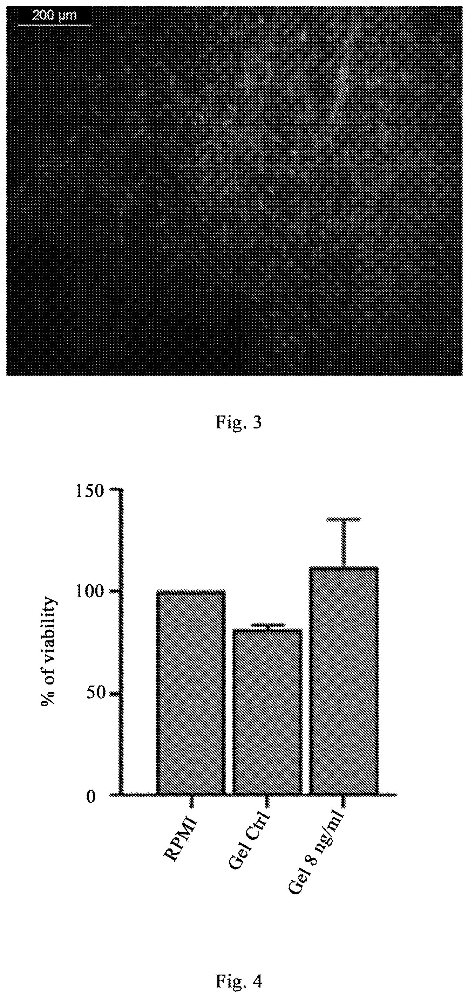

FIGURE 3 shows an image at the microscope of a cell culture of murine myoblasts at 14 days of culture in the hydrogel. The cells are elongated and do not have morphological alterations nor signs of suffering.
FIGURE 4 shows a graph relative to the vitality of the monocytes which at 7 days of culture is higher when the monocytes are cultivated on hydrogel.

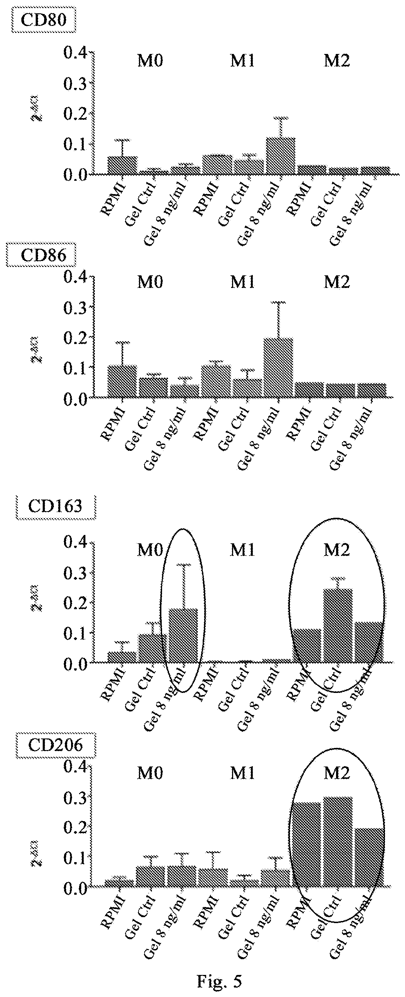

FIGURE 5 shows the polarization of the macrophages obtained from the isolation of monocytes from peripheral blood. The markers are differentiated towards regenerative phenotype M2 (CD163 and CD206) and are expressed in a significantly higher manner when the macrophages are cultivated on the hydrogel. In particular the polarization towards M2 also occurs spontaneously when in the presence of the hydrogel and in the absence of specific stimuli for the polarization. (M0 conditions, top right graph of figure 5). The macrophage polarization markers towards M1 (inflammatory condition) CD80 and CD86 are never significantly up-regulated, not even when in the presence of pro-inflammatory stimuli (M1 conditions).

### Detailed description of the invention

The object of the present invention is a hydrogel based on collagen from bovine pericardium in a concentration comprised between 0.3% and 0.8%, elastin, laminin, fibronectin and possibly crosslinking agents such as hexamethylene diisocyanate (HMDI) at concentrations variable between 0.01 and 0.05% with respect to the solid content of the material via intra-articular infiltrations with the object of reducing the inflammation and regenerating the lesioned tissue.

The product is a hydrogel which gels at body temperature (37°C), constituted by enzymatically-hydrolyzed extracellular matrix. The percentage of ECM in the hydrogel can vary from 0.3% to 0.8%. The ECM in turn is 95% composed of collagen, with the remaining 5% composed of other components, in particular elastin and decorin, useful for the functional regeneration of the matrix.

In order to reinforce the structure of the hydrogel, and prevent the disintegration thereof due to the breakage of the weak interactions between its components, it is possible to use crosslinking agents, such as hexamethylene diisocyanate (HMDI). Typically, but not exclusively, said agent is added to concentrations variable between 0.01 and 0.1% by weight with respect to the solid content. The addition of the crosslinking agent stabilizes the gelatinous matrix and prolongs the dissolution times thereof in the fluids. Based on the use needs, it is also possible to enrich the matrix with bioactive molecules such as antibiotics, growth factors, etc. which are trapped in the gel. It is then possible to bond substances which facilitate cell recruitment, also through the suitable modulation of the inflammatory response in order to speed up the colonization of the hydrogel and hence the formation of new ECM which will lead to the regeneration of the damaged tissue. The hydrogel, in fact, possesses a particular immunomodulatory capacity capable of inducing the polarization of the macrophages resident in the tissues from the inflammatory form M1 to the regenerative form M2 with the object of facilitating the tissue regeneration.

In addition, this hydro-collagen is adapted to be mixed with platelet-rich plasma or autologous cell therapy.

### Bibliography

*1. Kopeček J.* Hydrogel biomaterials: A smart future? Biomaterials. 2007;28(34):5185-5192. doi:10.1016/j.biomaterials. 2007. 07. 044
*2.* Francis L, Greco KV, Boccaccini AR, et al. Development of a novel hybrid bioactive hydrogel for future clinical applications. J Biomater Appl. 2018;33(3):447-465. doi:10.117710885328218794163
*3.* Kanikkannan N. Iontophoresis-Based Transdermal Delivery Systems: BioDrugs. 2002;16(5):339-347. doi:10.2165100063030-200216050-00003
*4.* Caló E, Khutoryanskiy VV. Biomedical applications of hydrogels: A review of patents and commercial products. Eur Polym J. 2015;65:252-267. doi:10.1016/j.eurpolymj.2014.11.024
*5.* Tibbitt MW, Anseth KS. Hydrogels as extracellular matrix mimics for 3D cell culture. Biotechnol Bioeng. 2009; 103(4):655-663. doi:10.1002/bit.22361
*6.* Biomedical Engineering - Frontiers and Challenges. InTech; 2011*.*
*7.* Morris AH, Stamer DK, Kyriakides TR. The host response to naturally-derived extracellular matrix biomaterials. Semin Immunol. 2017;29:72-91. doi:10.1016/j.smim.2017.01.002
*8. Kawecki M,* 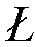 *abuś W, Klama-Baryla A, Kitala D, Kraut M, Glik J, et al.* A review of decellurization methods caused by an urgent need for quality control of cell-free extracellular matrix' scaffolds and their role in regenerative medicine. J Biomed Mater Res Part B Appl Biomater. 2018;106:909-23
*9.* Catoira MC, Fusaro L, Di Francesco D, Ramella M, Boccafoschi F. Overview of natural hydrogels for regenerative medicine applications. J Mater Sci Mater Med. 2019 Oct 10;30(10):115. doi: 10.1007/s10856-019-6318-7*.*
*10.* Zhang, Y. & Jordan, J. M. Epidemiology of osteoarthritis. Clinics in Geriatric Medicine 26, 355-369 (2010*).*
*11.* Xie, F. et al. Economic and Humanistic Burden of Osteoarthritis: A Systematic Review of Large Sample Studies. Pharmacoeconomics 34, 1087-1100 (2016*).*
*12.* Mobasheri, A., Bay-Jensen, A. C., van Spil, W. E., Larkin, J. & Levesque, M. C. Osteoarthritis Year in Review 2016: biomarkers (biochemical markers). Osteoarthritis and Cartilage 25, 199-208 (2017*).*
*13.* Liu, B., Zhang, M., Zhao, J., Zheng, M. & Yang, H. Imbalance of M1/M2 macrophages is linked to severity level of knee osteoarthritis. Exp. Ther. Med. 16, 5009-5014 (2018*).*
*14.* Fahy, N. et al. Human osteoarthritic synovium impacts chondrogenic differentiation of mesenchymal stem cells via macrophage polarisation state. Osteoarthr. Cartil. 22, 1167-1175 (2014*).*
*15.* Anderson, J. M., Rodriguez, A. & Chang, D. T. Foreign body reaction to biomaterials. Seminars in Immunology 20, 86-100 (2008*).*
*16.* Farhat, W. et al. Hydrogels for Advanced Stem Cell Therapies: A Biomimetic Materials Approach for Enhancing Natural Tissue Function. IEEE Rev. Biomed. Eng. 12, 333-351 (2018*).*

## Claims

1. Process for obtaining a hydrogel based on collagen from bovine pericardium, said process providing that:
- said hydrogel is produced starting from decellularized and lyophilized pericardium;
- it is subsequently finely divided in a manner so as to reduce the dimensions thereof;
- it is enzymatically digested with pepsin in acidic solution up to obtaining a pregel;
- said pregel is treated with water for the obtainment of the hydrogel;
- the hydrogel thus obtained is enriched with a crosslinking agent at a concentration comprised between 0.01% and 0.1% by weight with respect to the content of the solid, said crosslinking agent being hexamethylene diisocyanate.

2. Process according to the preceding claim **wherein** it is further provided to enrich the hydrogel with bioactive molecules selected from among antibiotics and growth factors.

3. Hydrogel based on collagen from decellularized bovine pericardium obtained according to any one of claims 1-2, said hydrogel comprising an enzymatically-hydrolyzed extracellular matrix in a quantity comprised between 0.3% and 0.8% by weight, elastin, laminin, fibronectin, the completion up to 100% by weight occurring with water, said enzymatically-hydrolyzed extracellular matrix comprising collagen at 95% by weight.

4. Hydrogel based on collagen from decellularized bovine pericardium according to the preceding claim **wherein** a crosslinking agent is further comprised, the latter being hexamethylene diisocyanate in a concentration comprised between 0.01% and 0.05% by weight.

5. Hydrogel based on decellularized bovine pericardium according to the preceding claim **wherein** bioactive molecules selected from among growth factors and antibiotics are further present.

6. Hydrogel based on collagen from decellularized bovine pericardium according to any one of the claims 3 - 5 for use in an osteoarthritis treatment method.

7. Hydrogel based on collagen from bovine pericardium according to any one of the claims 3 - 5 for use in an esthetic surgery treatment method.

8. Hydrogel based on collagen from bovine pericardium for use according to the preceding claim **wherein** the esthetic treatment is in the maxillofacial field.

9. Hydrogel based on collagen from bovine pericardium according to any one of the claims 3 - 5 for use in a method for treating skin ulcers.

10. Hydrogel based on collagen from bovine pericardium for use according to the preceding claim **wherein** the skin ulcers are those from diabetic foot.

## Patentansprüche

1. Verfahren zum Gewinnen eines Hydrogels basierend auf Kollagen aus Rinderperikardium, wobei das Verfahren vorsieht, dass::
- das Hydrogel ausgehend von dezellularisiertem und gefriergetrocknetem Perikardiumium hergestellt wird;
- es nachfolgend so fein zerteilt wird, dass seine Abmessungen verringert werden;
- es enzymatisch mit Pepsin in saurer Lösung aufbereitet wird, bis man ein Pre-Gel erhält;
- das Pre-Gel mit Wasser behandelt wird, um das Hydrogel zu erhalten;
- das so erhaltene Hydrogel mit einem Vernetzungsmittel mit einer Konzentration, die in Bezug auf den Feststoffgehalt zwischen 0,01 Gew.-% und 0,1 Gew.-% aufweist, angereichert wird, wobei das Vernetzungsmittel Hexamethylendiisocyanat ist.

2. Verfahren nach dem vorstehenden Anspruch**, wobei** es weiterhin vorgesehen ist, das Hydrogel mit bioaktiven Molekülen, die aus der Gruppe der Antibiotika und Wachstumsfaktoren ausgewählt sind, anzureichern.

3. Hydrogel basierend auf Kollagen aus dezellularisiertem Rinderperikardium, das gemäß einem der Ansprüche 1-2 gewonnen wurde, wobei das Hydrogel eine enzymatisch hydrolysierte extrazelluläre Matrix in einer Menge zwischen 0,3 Gew.-% und 0,8 Gew.-%, Elastin, Laminin, Fibronektin, die Vervollständigung bis auf 100 Gew.-% mit Wasser erfolgt, wobei die enzymatisch hydrolysierte extrazelluläre Matrix Kollagen mit 95 Gew.-% aufweist.

4. Hydrogel basierend auf Kollagen aus dezellularisiertem Rinderperikardium gemäß dem vorstehenden Anspruch**, wobei** weiterhin ein Vernetzungsmittel enthalten ist, wobei letzteres Hexamethylendiisocyanat in einer Konzentration, die zwischen 0,01 Gew.-% und 0,05 Gew.-% aufweist, ist.

5. Hydrogel basierend auf Kollagen aus dezellularisiertem Rinderperikardium gemäß dem vorstehenden Anspruch**, wobei** weiterhin bioaktive Moleküle, die aus Wachstumsfaktoren und Antibiotika ausgewählt sind, vorhanden sind.

6. Hydrogel basierend auf Kollagen aus dezellularisiertem Rinderperikardium gemäß einem der Ansprüche 3 - 5 zur Verwendung in einem Verfahren zur Behandlung von Osteoarthritis.

7. Hydrogel basierend auf Kollagen aus Rinderperikardium gemäß einem der Ansprüche 3 - 5 zur Verwendung in einem Behandlungsverfahren der ästhetischen Chirurgie.

8. Hydrogel basierend auf Kollagen aus Rinderperikardium zur Verwendung gemäß dem vorstehenden Anspruch**, wobei** die ästhetische Behandlung im maxillofazialen Bereich erfolgt.

9. Hydrogel basierend auf Kollagen aus Rinderperikardium gemäß einem der Ansprüche 3 - 5 zur Verwendung in einem Verfahren zur Behandlung von Hautgeschwüren.

10. Hydrogel basierend auf Kollagen aus Rinderperikardium zur Verwendung gemäß dem vorstehenden Anspruch**, wobei** es sich bei den Hautgeschwüren um solche des diabetischen Fußes handelt.

## Revendications

1. Procédé pour obtenir un hydrogel à base de collagène provenant de péricarde bovin, ledit procédé prévoyant que :
- ledit hydrogel est produit à partir d'un péricarde décellularisé et lyophilisé ;
- il est ensuite finement divisé de manière à en réduire les dimensions ;
- il est digéré par voie enzymatique avec de la pepsine en solution acide jusqu'à l'obtention d'un prégel ;
- ledit prégel est traité avec de l'eau pour l'obtention de l'hydrogel ;
- l'hydrogel ainsi obtenu est enrichi d'un agent de réticulation à une concentration comprise entre 0,01 % et 0,1 % en poids par rapport à la teneur du solide, ledit agent de réticulation étant le diisocyanate d'hexaméthylène.

2. Procédé selon la revendication précédente **dans lequel** il est en outre prévu d'enrichir l'hydrogel avec des molécules bioactives sélectionnées parmi les antibiotiques et les facteurs de croissance.

3. Hydrogel à base de collagène provenant de péricarde bovin décellularisé obtenu selon l'une quelconque des revendications 1 à 2, ledit hydrogel comprenant une matrice extracellulaire hydrolysée par voie enzymatique dans une quantité comprise entre 0,3 % et 0,8 % en poids, de l'élastine, de la laminine, de la fibronectine, l'action de compléter jusqu'à 100 % en poids se produisant avec de l'eau, ladite matrice extracellulaire hydrolysée par voie enzymatique comprenant du collagène à 95 % en poids.

4. Hydrogel à base de collagène provenant de péricarde bovin décellularisé selon la revendication précédente **dans lequel** un agent de réticulation est en outre compris, ce dernier étant le diisocyanate d'hexaméthylène dans une concentration comprise entre 0,01 % et 0,05 % en poids.

5. Hydrogel à base de péricarde bovin décellularisé selon la revendication précédente **dans lequel** des molécules bioactives sélectionnées parmi des facteurs de croissance et des antibiotiques sont en outre présentes.

6. Hydrogel à base de collagène provenant de péricarde bovin décellularisé selon l'une des revendications 3 à 5 pour une utilisation dans une méthode de traitement de l'arthrose.

7. Hydrogel à base de collagène provenant de péricarde bovin selon l'une quelconque des revendications 3 à 5 pour une utilisation dans une méthode de traitement de chirurgie esthétique.

8. Hydrogel à base de collagène provenant de péricarde bovin pour une utilisation selon la revendication précédente **dans lequel** le traitement esthétique se situe dans le domaine maxillo-facial.

9. Hydrogel à base de collagène provenant de péricarde bovin selon l'une quelconque des revendications 3 à 5 pour une utilisation dans une méthode pour traiter des ulcères cutanés.

10. Hydrogel à base de collagène provenant de péricarde bovin pour une utilisation selon la revendication précédente **dans lequel** les ulcères cutanés sont ceux du pied diabétique.
